**Europäisches Patentamt**

**European Patent Office**

**Office européen des brevets**

(19)

(11) Veröffentlichungsnummer: **0 091 537**
**B1**

(12) ## EUROPÄISCHE PATENTSCHRIFT

(45) Veröffentlichungstag der Patentschrift:
08.01.86

(51) Int. Cl.⁴: **C 01 B 33/28, B 01 J 29/28**

(21) Anmeldenummer: **83100047.6**

(22) Anmeldetag: **05.01.83**

(54) **Kristallines Alumosilikat, Verfahren zu seiner Herstellung und Verwendung.**

(30) Priorität: **27.03.82 DE 3211433**

(43) Veröffentlichungstag der Anmeldung:
**19.10.83 Patentblatt 83/42**

(45) Bekanntmachung des Hinweises auf die Patenterteilung:
**08.01.86 Patentblatt 86/2**

(84) Benannte Vertragsstaaten:
**AT BE CH DE FR GB IT LI LU NL SE**

(56) Entgegenhaltungen:
**US - A - 3 947 482**
**US - A - 4 285 922**

(73) Patentinhaber: **Degussa Aktiengesellschaft,**
**Weissfrauenstrasse 9, D-6000 Frankfurt am Main 1 (DE)**

(72) Erfinder: **Baacke, Michael, Dr., Grünaustrasse 19,**
**D-6450 Hanau 9 (DE)**
Erfinder: **Kleinschmit, Peter, Dr., Wildaustrasse 19,**
**D-6450 Hanau 9 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung des neuen kristallinen Alumosilikates PZ 1, das Alumosilikat PZ 1 selbst, die Herstellung des Alumosilikates PZ 2 aus dem Alumosilikat PZ 1, das Alumosilikat PZ 2 selbst, sowie die Verwendung des Alumosilikates PZ 2 als Katalysator.

Gegenstand der Erfindung ist ein kristallines Alumosilikat PZ 1 mit der chemischen Zusammensetzung:

$$3{,}0 \pm 1{,}0\% \ Na_2O$$
$$1{,}5 \pm 1{,}0\% \ Al_2O_3$$
$$85{,}0 \pm 5{,}0\% \ SiO_2$$
$$0{,}5 \pm 0{,}3\% \ P_2O_5$$
$$5{,}0 \pm 3{,}0\% \ \text{Glühverlust}$$

und dem nachstehenden Röntgenbeugungsdiagramm:

| d [Å] | I/I₀ |
|---|---|
| 15,49 | 100 |
| 9,97 | 7 |
| 9,18 | 1 |
| 7,31 | 2 |
| 5,15 | 17 |
| 4,84 | 13 |
| 4,55 | 7 |
| 4,23 | 5 |
| 3,68 | 6 |
| 3,56 | 14 |
| 3,44 | 59 |
| 3,30 | 30 |
| 3,15 | 28 |
| 2,15 | 18 |
| 1,88 | 3 |
| 1,83 | 6 |

Das erfindungsgemässe Alumosilikat PZ 1 kann gemäss einem weiteren Gegenstand der Erfindung hergestellt werden, indem man die Kristallisation aus einer wässerigen $SiO_2$, $Al_2O_3$ und alkali-haltigen Synthesemischung in Gegenwart einer organischen Templatverbindung der allgemeinen Formel $R_3P-(CH_2)_n-PR_3X_2$, in der R = Methyl-, Äthyl-, Propyl- und/oder Butyl-, n = 2, 3, 4, 5 und/oder 6 und X = Br⊖ und/oder J⊖ bedeuten, durchführt, wobei man die Synthesemischung mit der molaren Zusammensetzung:

$$SiO_2/Al_2O_3 = 20\text{-}250$$
$$Na_2O/SiO_2 = 0{,}01\text{-}0{,}5$$
$$OH^\ominus/SiO_2 = 0{,}01\text{-}1{,}0$$
$$R/SiO_2 = 0{,}01\text{-}0{,}2$$
$$H_2O/SiO_2 = 10\text{-}40$$

bei Temperaturen zwischen 90 und 200°C bei autogenem Druck solange rührt, bis Kristalle gebildet sind, die Kristalle abtrennt, wäscht und trocknet.

In einer bevorzugten Ausführungsform der Erfindung kann die Templatverbindung $Bu_3P-(CH_2)_3-PBu_3Br_2$ und/oder $Bu_3P-(CH_2)_3-PBu_3J_2$ eingesetzt werden.

Das mittels dem erfindungsgemässen Verfahren hergestellte Alumosilikat trägt die Bezeichnung PZ 1. Es kann durch seine Zusammensetzung, sein Adsorptionsverhalten, seine Kristallform und sein Röntgenbeugungsdiagramm charakterisiert werden.

Das Alumosilikat PZ 1 weist die folgende chemische Zusammensetzung auf:

$$3{,}0 \pm 1{,}0\% \ Na_2O$$
$$1{,}5 \pm 1{,}0\% \ Al_2O_3$$
$$85{,}0 \pm 5{,}0\% \ SiO_2$$
$$0{,}5 \pm 0{,}3\% \ P_2O_5$$
$$5{,}0 \pm 3{,}0\% \ \text{Glühverlust}$$

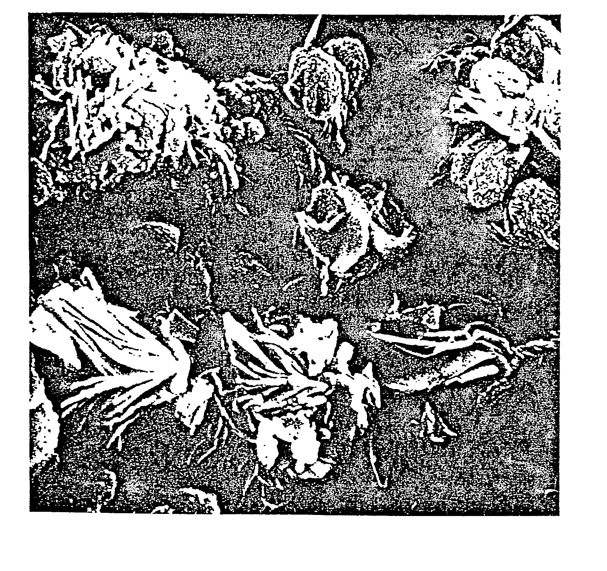

Rasterelektronenmikroskopaufnahmen zeigen zu Agglomeraten zusammengewachsene Plättchen (vgl. Fig. 1).

Das Röntgenbeugungsdiagramm (Tabelle 1) zeigt im wesentlichen folgende Linien:

| d [Å] | I/I₀ |
|---|---|
| 15,49 | 100 |
| 9,97 | 7 |
| 9,18 | 1 |
| 7,31 | 2 |
| 5,15 | 17 |
| 4,84 | 13 |
| 4,55 | 7 |
| 4,23 | 5 |
| 3,68 | 6 |
| 3,56 | 14 |
| 3,44 | 59 |
| 3,30 | 30 |
| 3,15 | 28 |
| 2,15 | 18 |
| 1,88 | 3 |
| 1,83 | 6 |

Es wurde nach Standardmethoden mit einem Szintillationszähler-Diffraktometer bei Cu-$K_{\alpha1}$-Strahlung gemessen. d [Å] bezeichnet die interplanaren Abstände und I/I deren relative Intensitäten.

Adsorptionsmessungen (20°C, 50% relativer Dampfdruck) führten zu folgenden Ergebnissen (Angaben in g adsorbierte Substanz pro g Silikat):

n-Hexan 0,01
Benzol 0,03
Wasser 0,07

Wird das Alumosilikat PZ 1 mit verdünnten Säuren, vorzugsweise Mineralsäuren, wie beispielsweise 1n Schwefelsäure oder Salzsäure behandelt bzw. einem Ionenaustausch mit $NH_4Cl$ und nachfolgender Calzinierung unterworfen, so entsteht ein neues kristallines Alumosilikat PZ 2.

Dementsprechend ist ein weiterer Gegenstand der Erfindung ein kristallines Alumosilikat PZ 2 mit der chemischen Zusammensetzung:

$$0{,}05 \pm 0{,}03\% \ Na_2O$$
$$1{,}40 \pm 0{,}6 \ \% \ Al_2O_3$$

88,0 ±3,0 % $SiO_2$
0,5 ±0,2 % $P_2O_5$
3,0 ±1,0 % Glühverlust

und dem nachstehenden Röntgenbeugungsdiagramm

| d [Å] | $I/I_0$ |
|-------|---------|
| 13,48 | 100 |
| 7,34 | 24 |
| 3,55 | 55 |
| 3,40 | 85 |
| 1,85 | 20 |

Rasterelektronenmikroskopaufnahmen lassen dagegen keine Änderung von Kristallform oder -grösse beim Übergang PZ 1 → PZ 2 erkennen.

Adsorptionsversuche bei 20°C und 50% relativen Dampfdruckes führten zu folgenden Ergebnissen:

n-Hexan 0,06
Benzol 0,04
Wasser 0,05

(Angaben in g adsorbierter Substanz pro g Silikat).

Das erfindungsgemässe Alumosilikat PZ 2 kann gemäss einem weiteren Gegenstand der Erfindung hergestellt werden, indem man die Kristallisation aus einer wässerigen $SiO_2$, $Al_2O_3$ und alkali-haltigen Synthesemischung in Gegenwart einer organischen Templatverbindung der allgemeinen Formel $R_3P-(CH_2)_n-PR_3X_2$, in der R = Methyl-, Äthyl-, Propyl- und/oder Butyl-, n = 2, 3, 4, 5 und/oder 6 und X = $Br^\ominus$ und/oder $J^\ominus$ bedeuten, durchführt, wobei man die Synthesemischung mit der molaren Zusammensetzung:

$SiO_2/Al_2O_3$ = 20-250
$Na_2O/SiO_2$ = 0,01-0,5
$OH^\ominus/SiO_2$ = 0,01-1,0
$R/SiO_2$ = 0,01-0,2
$H_2O/SiO_2$ = 10-40

bei Temperaturen zwischen 90 und 200°C bei autogenem Druck solange rührt, bis Kristalle gebildet sind, die Kristalle abtrennt, wäscht, trocknet und anschliessend mit verdünnten Säuren behandelt oder einem Ionenaustausch mit $NH_4Cl$ unterwirft und anschliessend calziniert.

Das Alumosilikat PZ 2 ist zur Verwendung als Katalysatoren, insbesondere zur Umwandlung von Alkoholen in Kohlenwasserstoffe geeignet.

Aus der Literatur (HP. Eugster, Science *57*, 1157 (1967)) ist ein mineralisches Schichtsilikat bekannt, dessen Röntgenbeugungsdiagramm dem von PZ 1 ähnelt, jedoch bei vielen interplanaren Abständen deutliche Unterschiede aufweist.

Das mineralische Schichtsilikat, Magadiit, wird als Natriumsilikat-Hydrat der Formel $NaSi_7O_{13}(OH)_3 \cdot 3H_2O$ beschrieben, während zur Kristallisation von PZ 1 Aluminiumverbindungen anwesend sein müssen. Bei Erhöhung des $SiO_2/Al_2O_3$-Verhältnisses über etwa 250 hinaus wird nicht mehr PZ 1 sondern ein anderes Produkt erhalten.

In der gleichen Literaturstelle wird auch die Umwandlung von Magadiit in eine Verbindung SH beschrieben, deren Röntgenbeugungsdiagramm dem von PZ 2 ähnelt, jedoch ebenso deutliche Unterschiede aufweist. Ausserdem wird in der oben angeführten Literaturstelle ausführlich die Überführung von SH in Magadiit durch Behandeln mit $Na_2CO_3$ oder NaOH beschrieben. Bei der Behandlung von PZ 2 mit diesen Verbindungen wird analytisch zwar eine Natrium-Aufnahme nachgewiesen, es kommt jedoch nicht zu einer Strukturänderung PZ 2→PZ 1, wie durch die Aufnahmen von Röntgenbeugungsdiagramm leicht nachgewiesen werden kann.

Aus der US-PS Nr. 4285922 ist die Herstellung eines nichtidentifizierten Materials, das ein ähnliches Röntgenbeugungsdiagramm wie PZ 1 aufweist, als Nebenprodukt bei der Kristallisation von ZSM 5 bekannt. Dieses Material entsteht jedoch bevorzugt bei $SiO_2/Al_2O_3$-Verhältnissen, die grösser als 500, mindestens jedoch grösser als 300 sind. PZ 1 dagegen entsteht bei Molverhältnissen zwischen etwa 50 und etwa 250. Ein weiterer, entscheidender Unterschied besteht im Verhalten bei Ionenaustausch. Das in der US-PS Nr. 4285922 angegebene Röntgenbeugungsdiagramm ist charakteristisch für alle durch Ionenaustausch erhaltenen Produkte, während es bei PZ 1 beim Ionenaustausch zum Beispiel mit $NH_4Cl$ zu einer Umlagerung zu PZ 2 kommt, für das ein verändertes Röntgenbeugungsdiagramm beobachtet wird.

*Beispiel 1:*

2,5 g Tonerdetrihydrat werden in 50 ml einer 6,2N NaOH gelöst und zu einer Suspension von 120 g gefällter Kieselsäure (89% $SiO_2$) in einer Lösung von 25 g $Bu_3P-(CH_2)_3-PBu_3Br_2$ in 750 ml $H_2O$ gegeben. Die Mischung wird 20 min bei 50°C gerührt, in einen Stahlautoklaven überführt und 310 h bei 100°C gerührt. Nach beendeter Kristallisation werden die Kristalle durch Filtration abgetrennt, mit Wasser gewaschen und 12 h bei 120°C getrocknet.

*Beispiel 2:*

Die Durchführung geschieht analog zu Beispiel 1, jedoch wird anstelle von Tonerdetrihydrat die gleiche Menge Natriumaluminat eingesetzt, die NaOH-Menge auf 3,5 g verringert und 200 h bei 130°C gerührt.

*Beispiel 3:*

Hier werden Unterschied zu Beispiel 1 4,2 g Tonerdetrihydrat, 16,5 g NaOH und 100 g gefällter Kieselsäure verwendet und 50 h bei 160°C gerührt.

*Beispiel 4:*

Die Mengen betragen hier:

6,5 g Tonerdetrihydrat
25 g NaOH
310 g gefällte Kieselsäure
60 g $Bu_3P-(CH_2)_3-PBu_3Br_2$
2500 ml $H_2O$

Es wird 14 h bei 175°C kristallisiert.

In den Beispielen 1 bis 4 wird gut kristallisiertes Alumosilikat PZ 1 erhalten.

*Beispiel 5:*

50 g PZ 1 aus Beispiel 2 werden in 500 ml 1 N HCl 2 h bei 80°C gerührt, abfiltriert, mit $H_2O$ bis zur Neutralität gewaschen und 17 h bei 120°C getrocknet.

*Beispiel 6:*

30 g PZ 1 aus Beispiel 2 werden in 300 ml 5N $NH_4Cl$-Lösung 2 h bei 80°C gerührt, abfiltriert, mit $H_2O$ bis zur $Cl^\ominus$-Freiheit gewaschen, 4 h bei 120°C getrocknet und 16 h bei 550°C calziniert.

Die Beispiele 5 und 6 ergeben gut kristallisiertes Alumosilikat PZ 2.

## Patentansprüche

1. Kristallines Alumosilikat PZ 1 mit der chemischen Zusammensetzung

$3,0 \pm 1,0\%$ $Na_2O$
$1,5 \pm 1,0\%$ $Al_2O_3$
$85,0 \pm 5,0\%$ $SiO_2$
$0,5 \pm 0,3\%$ $P_2O_5$
$5,0 \pm 3,0\%$ Glühverlust

und dem nachstehenden Röntgenbeugungsdiagramm:

| d [Å] | I/I$_o$ |
|---|---|
| 15,49 | 100 |
| 9,97 | 7 |
| 9,18 | 1 |
| 7,31 | 2 |
| 5,15 | 17 |
| 4,84 | 13 |
| 4,55 | 7 |
| 4,23 | 5 |
| 3,68 | 6 |
| 3,56 | 14 |
| 3,44 | 59 |
| 3,30 | 30 |
| 3,15 | 28 |
| 2,15 | 18 |
| 1,88 | 3 |
| 1,83 | 6 |

2. Verfahren zur Herstellung des kristallinen Alumosilikates PZ 1 gemäss Anspruch 1, dadurch gekennzeichnet, dass man die Kristallisation aus einer wässerigen $SiO_2$, $Al_2O_3$ und alkali-haltigen Synthesemischung in Gegenwart einer organischen Templatverbindung der allgemeinen Formel $R_3P-(CH_2)_n-PR_3X_2$, in der R = Methyl-, Äthyl-, Propyl- und/oder Butyl-, n = 2, 3, 4, 5 und/oder 6 und X = $Br^\ominus$ und/oder $J^\ominus$ bedeuten, durchführt, wobei man die Synthesemischung mit der molaren Zusammensetzung:

$SiO_2/Al_2O_3 = 20\text{-}250$

$Na_2O/SiO_2 = 0,01\text{-}0,5$
$OH^\ominus/SiO_2 = 0,01\text{-}1,0$
$R/SiO_2 = 0,01\text{-}0,2$
$H_2O/SiO_2 = 10\text{-}40$

bei Temperaturen zwischen 90 und 200°C bei autogenem Druck solange rührt, bis Kristalle gebildet sind, die Kristalle abtrennt, wäscht und trocknet.

3. Verwendung des kristallinen Alumosilikates PZ 1 gemäss Anspruch 1 als Zwischenprodukt zur Herstellung des Alumosilikates PZ 2.

4. Kristallines Alumosilikat PZ 2 mit der chemischen Zusammensetzung:

$0,05 \pm 0,03\%$ $Na_2O$
$1,40 \pm 0,6$ % $Al_2O_3$
$88,0 \pm 3,0$ % $SiO_2$
$0,5 \pm 0,2$ % $P_2O_5$
$3,0 \pm 1,0$ % Glühverlust

und dem nachstehenden Röntgenbeugungsdiagramm:

| d [Å] | I/I$_o$ |
|---|---|
| 13,48 | 100 |
| 7,34 | 24 |
| 3,55 | 55 |
| 3,40 | 85 |
| 1,85 | 20 |

5. Verfahren zur Herstellung des Alumosilikates PZ 2 gemäss Anspruch 4, dadurch gekennzeichnet, dass man die Kristallisation aus einer wässerigen $SiO_2$, $Al_2O_3$ und alkali-haltigen Synthesemischung in Gegenwart einer organischen Templatverbindung der allgemeinen Formel $R_3P-(CH_2)_n-PR_3X_2$, in der R = Methyl-, Äthyl-, Propyl- und/oder Butyl-, n = 2, 3, 4, 5 und/oder 6 und X = $Br^\ominus$ und/oder $J^\ominus$ bedeuten, durchführt, wobei man die Synthesemischung mit der molaren Zusammensetzung:

$SiO_2/Al_2O_3 = 20\text{-}250$
$Na_2O/SiO_2 = 0,01\text{-}0,5$
$OH^\ominus/SiO_2 = 0,01\text{-}1,0$
$R/SiO_2 = 0,01\text{-}0,2$
$H_2O/SiO_2 = 10\text{-}40$

bei Temperaturen zwischen 90 und 200°C bei autogenem Druck solange rührt, bis Kristalle gebildet sind, die Kristalle abtrennt, wäscht, trocknet und anschliessend mit verdünnten Säuren behandelt oder einem Ionenaustausch mit $NH_4Cl$ unterwirft und anschliessend calziniert.

6. Verwendung des kristallinen Alumosilikates PZ 2 gemäss Anspruch 4 als Katalysator zur Umwandlung von Alkoholen in Kohlenwasserstoffe.

## Claims

1. Crystalline aluminium silicate PZ 1 with the chemical composition:

$3.0 \pm 1.0\%$ $Na_2O$

1.5±1.0% $Al_2O_3$
85.0±5.0% $SiO_2$
0.5±0.3% $P_2O_5$
5.0±3.0% annealing loss

and with the following X-ray diffraction pattern:

| d [Å] | I/I$_o$ |
|-------|---------|
| 15.49 | 100 |
| 9.97 | 7 |
| 9.18 | 1 |
| 7.31 | 2 |
| 5.15 | 17 |
| 4.84 | 13 |
| 4.55 | 7 |
| 4.23 | 5 |
| 3.68 | 6 |
| 3.56 | 14 |
| 3.44 | 59 |
| 3.30 | 30 |
| 3.15 | 28 |
| 2.15 | 18 |
| 1.88 | 3 |
| 1.83 | 6 |

2. A process for the production of crystalline aluminium silicate PZ 1 according to Claim 1, characterised in that the crystallization is carried out from an aqueous $SiO_2$, $Al_2O_3$ and alkali-containing synthesis mixture in the presence of an organic template compound of the general formula $R_3P-(CH_2)_n-PR_3X_2$, wherein R = methyl-, ethyl-, propyl- and/or butyl- n = 2, 3, 4, 5 and/or 6 and X = $Br^\ominus$ and/or $I^\ominus$, the synthesis mixture with the molar composition:

$$SiO_2/Al_2O_3 = 20\text{-}250$$
$$Na_2O/SiO_2 = 0.01\text{-}0.5$$
$$OH^\ominus/SiO_2 = 0.01\text{-}1.0$$
$$R/SiO_2 = 0.01\text{-}0.2$$
$$H_2O/SiO_2 = 10\text{-}40$$

being stirred at a temperature of from 90 to 200°C under autogenous pressure, until crystals are formed, the crystals being separated, washed and dried.

3. Use of the crystalline aluminium silicat PZ 1 according to Claim 1 as intermediate product for the production of the aluminium silicate PZ 2.

4. Crystalline aluminium silicate PZ 2 with the chemical composition:

0.05±0.03% $Na_2O$
1.40±0.6 % $Al_2O_3$
88.0 ±3.0 % $SiO_2$
0.5 ±0.2 % $P_2O_5$
3.0 ±1.0 % annealing loss

and with the following X-ray diffraction pattern:

| d [Å] | I/I$_o$ |
|-------|---------|
| 13.48 | 100 |
| 7.34 | 24 |
| 3.55 | 55 |
| 3.40 | 85 |
| 1.85 | 20 |

5. A process for the production of the aluminium silicate PZ 2 according to Claim 4, characterised in that the crystallization is carried out from an aqueous $SiO_2$, $Al_2O_3$ and alkali-containing synthesis mixture in the presence of an organic template compound of the general formula $R_3P-(CH_2)-PR_3X_2$, wherein R = methyl-, ethyl-, propyl- and/or butyl-, n = 2, 3, 4, 5 and/or 6 and X = $Br^\ominus$ and/or $I^\ominus$, the synthesis mixture with the molar composition:

$$SiO_2/Al_2O_3 = 20\text{-}250$$
$$Na_2O/SiO_2 = 0.01\text{-}0.5$$
$$OH^\ominus/SiO_2 = 0.01\text{-}1.0$$
$$R/SiO_2 = 0.01\text{-}0.2$$
$$H_2O/SiO_2 = 10\text{-}40$$

being stirred at a temperature of from 90 to 200°C under autogeneous pressure, until crystals are formed, the crystals being separated, washed, dried and subsequently treated with dilute acids or subjected to an ion exchange with $NH_4Cl$ and subsequently calcined.

6. Use of the crystalline aluminium silicate PZ 2 according to Claim 4 as catalyst for transforming alcohols into hydrocarbons.

**Revendications**

1. Silicate d'aluminium PZ 1 cristallisé ayant la composition chimique suivante:

3,0±1,0% $Na_2O$
1,5±1,0% $Al_2O_3$
85,0±5,0% $SiO_2$
0,5±0,3% $P_2O_5$
5,0±3,0% perte au feu,

et le diagramme de diffraction Röntgen ci-après:

| d [Å] | I/I$_o$ |
|-------|---------|
| 15,49 | 100 |
| 9,97 | 7 |
| 9,18 | 1 |
| 7,31 | 2 |
| 5,15 | 17 |
| 4,84 | 13 |
| 4,55 | 7 |
| 4,23 | 5 |
| 3,68 | 6 |
| 3,56 | 14 |
| 3,44 | 59 |
| 3,30 | 30 |
| 3,15 | 28 |
| 2,15 | 18 |
| 1,88 | 3 |
| 1,83 | 6 |

2. Procédé pour la fabrication du silicate d'aluminium PZ 1 de la revendication 1, caractérisé en ce qu'on procède à la cristallisation à partir d'un mélange de synthèse aqueux de $SiO_2$, de $Al_2O_3$, contenant un agent alcalin, en présence d'un composé «templat» organique dont la formule générale est $R_3P-(CH_2)_n-PR_3X_2$, où R représente un méthyl-, éthyl-, propyl- et/ou butyl-, n = 2, 3, 4, 5 et/ou 6, et $X = Br^{\ominus}$ et/ou $I^{\ominus}$ pendant que l'on agite le mélange de synthèse, dont la composition molaire est:

$$SiO_2/Al_2O_3 = 20\text{-}250$$
$$Na_2O/SiO_2 = 0,01\text{-}0,5$$
$$OH^{\ominus}/SiO_2 = 0,01\text{-}1,0$$
$$R/SiO_3 = 0,01\text{-}0,2$$
$$H_2O/SiO_2 = 10\text{-}40$$

à des températures entre 90 et 200°C sous la pression autogène, assez longtemps pour que les cristaux se forment, sépare ces cristaux, les lave et les sèche.

3. Utilisation du silicate d'aluminium PZ 1 suivant la revendication 1 comme produit intermédiaire pour la fabrication du silicate d'aluminium PZ 2.

4. Silicate d'aluminium PZ 2 ayant la composition chimique suivante:

$$0,05 \pm 0,03\% \ Na_2O$$
$$1,40 \pm 0,6 \ \% \ Al_2O_4$$
$$88,0 \ \pm 3,0 \ \% \ SiO_2$$
$$0,5 \ \pm 0,2 \ \% \ P_2O_5$$
$$3,0 \ \pm 1,0 \ \% \ \text{perte au feu}$$

et le diagramme de diffraction Röntgen ci-après:

| d [Å] | $I/I_o$ |
|-------|---------|
| 13,48 | 100 |
| 7,34 | 24 |
| 3,55 | 55 |
| 3,40 | 85 |
| 1,85 | 20 |

5. Procédé pour la fabrication du silicate d'aluminium PZ 2 suivant la revendication 4, caractérisé en ce que l'on procède à la cristallisation à partir d'un mélange de synthèse aqueux de $SiO_2$, de $Al_2O_3$, contenant un agent alcalin, en présence d'un composé «templat» organique dont la formule générale est $R_3P-(CH_2)_n-PR_3X_2$, où R représente un méthyl-, éthyl-, propyl- et/ou butyl-, n = 2, 3, 4, 5 et/ou 6 et $X = Br^{\ominus}$ et/ou $I^{\ominus}$ pendant que l'on agite le mélange de synthèse, dont la composition molaire est:

$$SiO_2/Al_2O_3 = 20\text{-}250$$
$$Na_2O/SiO_2 = 0,01\text{-}0,5$$
$$OH^{\ominus}/SiO_2 = 0,01\text{-}1,0$$
$$R/SiO_2 = 0,01\text{-}0,2$$
$$H_2O/SiO_2 = 10\text{-}40$$

à des températures entre 90 et 200°C sous la pression autogène, assez longtemps pour que les cristaux se forment, sépare ces cristaux, les lave, les sèche et ensuite les traite avec des acides dilués ou les soumet à un échange d'ions avec $NH_4Cl$ puis les calcine.

6. Utilisation du silicate d'aluminium PZ 2 suivant la revendication 4, comme catalyseur pour la transformation des alcools en hydrocarbures.